# EUROPEAN PATENT APPLICATION

(11) **EP 4 052 739 A2**
(43) Date of publication of application: **07.09.2022**
(21) Application number: 20882770.9
(22) Date of filing: 23.10.2020
(51) Int. Cl.: A61M 5/142, A61M 5/145, A61M 5/172

(54) **CARTRIDGE MODULE, LIQUID MEDICINE DISCHARGE ASSEMBLY, AND LIQUID MEDICINE INJECTION DEVICE COMPRISING SAME**

(30) Priority: 31.10.2019 KR 20190138204; 22.10.2020 KR 20200137625
(71) Applicant: Eoflow Co., Ltd., Bundang-gu, Seongnam-si Gyeonggi-do 13605 (KR)
(72) Inventor: KIM, Jesse Jaejin, Seongnam-si Gyeonggi-do 13562 (KR); HAN, Yongjoon, Seoul 05266 (KR)
(74) Representative: Mewburn Ellis LLP
(86) International application number: PCT/KR2020/014592
(87) International publication number: WO 2021/085953

(57) **Abstract**

A drug injection device includes a base body, a needle module including a needle injected into a user's body and discharging a drug and mounted on the base body, a cartridge module including a reservoir storing the drug discharged to the needle and having a structure attachable to and detachable from the base body, and a drug discharge assembly disposed between the needle and the reservoir and dispensing the drug to the needle in a preset amount.

## Description

### TECHNICAL FIELD

The present disclosure relates to a cartridge module, a drug discharge assembly and a drug injection device including the same.

### BACKGROUND ART

In general, drug injection devices such as insulin injection devices are used to inject drugs into patients' bodies. Although the drug injection devices are sometimes used by professional medical staff such as doctors or nurses, in most cases, it is used by general public such as the patients themselves or guardians. Diabetic patients, especially pediatric diabetic patients, need to inject drugs such as insulin into the body at regular intervals. Therefore, a patch-type drug injection device that is used by being attached to human body for a certain period of time, is being developed. This type of drug injection device may be used by being attached to the body such as the patient's abdomen or waist in a patch-type for a certain period of time.

In the case of being attached to the human body, drug injection devices need to have excellent wearability, ease of use, excellent durability, and low power consumption. In particular, since drug injection devices are attached to the patient's skin, the user should be able to simply and conveniently insert a needle or cannula into the patient's skin.

In the drug injection device, it is important that the drug be injected in a preset amount. In order to inject a drug in a preset amount, a device dispensing the drug in a preset amount is required inside the drug injection device. Research on dispensing and discharging drugs in preset amounts by mechanical methods is ongoing.

### DESCRIPTION OF EMBODIMENTS

### TECHNICAL PROBLEM

An objective of the present disclosure is to provide a cartridge module, a drug discharge assembly and a drug injection device including the same that a user may easily replace a drug.

### TECHNICAL SOLUTION TO PROBLEM

One embodiment of the present disclosure provides a drug injection device including a base body, a needle module including a needle injected into a user's body and discharging a drug and mounted on the base body, a cartridge module including a reservoir storing the drug discharged to the needle and having a structure attachable to and detachable from the base body, and a drug discharge assembly disposed between the needle and the reservoir and dispensing the drug to the needle in a preset amount.

### ADVANTAGEOUS EFFECTS OF DISCLOSURE

In a drug injection device according to the embodiments of the present disclosure, only a cartridge module may be replaced while leaving a body on which a needle is mounted, so that mounting time of the drug injection device may be extended, through which a user's inconvenience as well as pain caused by needle replacement may be reduced.

A drug discharge assembly and a drug injection device according to an embodiment of the present disclosure may inject a drug into a subject in a preset amount. When a drive shaft of a driving unit linearly reciprocates, a rotation angle of a rotation unit is adjusted, the amount of drug moved along a curve section is controlled, so that the drug may be discharged in a preset amount into a needle assembly.

The drug discharge assembly and the drug injection device according to an embodiment of the present disclosure distribute the drug multiple times, so that a small amount of the drug may be accurately discharged. Since a pressing portion of a pressing unit compresses a tube, a preset amount of drug is primarily dispensed between a pair of pressing points. Since the drug that is primarily dispensed is secondarily dispensed while moving the curve section, so that the drug discharge assembly accurately dispenses a small amount of drug, and the drug injection device may inject the drug into the subject in a preset amount.

The drug discharge assembly and the drug injection device according to an embodiment of the present disclosure may accurately dispense a drug with a simple driving mechanism. Even without providing an additional driving source to a reservoir, the rotation of the pressing unit allows the drug to move from the reservoir to the needle assembly. Since the drug discharge assembly functions as a driving source to move the drug while dispensing the drug, the structure of the drug injection device may be formed simply and compactly.

Of course, the scope of the present disclosure is not limited by these effects.

### BRIEF DESCRIPTION OF DRAWINGS

FIG. 1 is a perspective view illustrating a drug injection device according to an embodiment of the present disclosure.
FIG. 2 is a perspective view for describing an internal arrangement of the drug injection device of FIG. 1.
FIGS. 3 and 4 are views for describing a cartridge module of FIG. 2.
FIG. 5 is a view for describing a main body except the cartridge module in the drug injection device of FIG. 2.
FIG. 6 is a bottom view illustrating some parts of the main body of FIG. 5.
FIG. 7 is a view illustrating a state in which a connecting member of the cartridge module and a tube of a drug discharge assembly are connected.
FIG. 8 is a view for describing a method of using a drug injection device according to an embodiment of the present disclosure.
FIG. 9 is a perspective view illustrating a drug discharge assembly according to another embodiment of the present disclosure.
FIG. 10 is a plan view illustrating the drug discharge assembly of FIG. 9.
FIG. 11 is a cross-sectional view taken along IV-IV of FIG. 9.
FIGS. 12 and 13 are diagrams illustrating an operation of the drug discharge assembly.
FIG. 14 is a view illustrating a reservoir connected to the drug discharge assembly of FIG. 9.
FIG. 15 is a diagram illustrating a drug discharge assembly according to another embodiment of the present disclosure.

### MODE OF DISCLOSURE

One embodiment of the present disclosure provides a drug injection device including a base body, a needle module including a needle injected into a user's body and discharging a drug and mounted on the base body, a cartridge module including a reservoir storing the drug discharged to the needle and having a structure attachable to and detachable from the base body, and a drug discharge assembly disposed between the needle and the reservoir and dispensing the drug to the needle in a preset amount.

In one embodiment of the present disclosure, the drug injection device may further include a driving unit driving the drug discharge assembly, and the cartridge module may further include a battery supplying power to the driving unit.

In one embodiment of the present disclosure, the cartridge module may further include a case accommodating the reservoir and the battery as one body.

In one embodiment of the present disclosure, the cartridge module may further include a connecting member fluidly connecting the reservoir and the drug discharge assembly so that the drug in the reservoir flows to the drug discharge assembly when the cartridge module is mounted on the base body.

In one embodiment of the present disclosure, the drug discharge assembly may include an insertion portion inserted into the connecting member, and the connecting member may include a barrier rib that is penetrable by the insertion portion.

One embodiment of the present disclosure provides a cartridge module that is replaceably mounted on a drug injection device including a base body, a needle module including a needle discharging a drug injected into a user's body and mounted on the base body, and a drug discharge assembly dispensing the drug to the needle in a preset amount, and that includes a reservoir storing the drug discharged to the needle, and a case having a structure accommodating the reservoir therein and attachable to and detachable from the base body.

In one embodiment of the present disclosure, the cartridge module may further include a battery supplying power to a driving unit driving the drug discharge assembly, and the case may accommodate the reservoir and the battery as one body.

In one embodiment of the present disclosure, the cartridge module may further include a connecting member fluidly connecting the reservoir and the drug discharge assembly so that the drug in the reservoir flows to the drug discharge assembly when the cartridge module is mounted on the base body.

In one embodiment of the present disclosure, the connecting member may include a barrier rib penetrable by an insertion portion of the drug discharge assembly.

Other aspects, features, and advantages other than those described above will become apparent from the following detailed description, claims and drawings for carrying out the following disclosure.

### MODE FOR INVENTION

Since the present disclosure can undergo various transformations and can have various embodiments, specific embodiments are illustrated in the drawings and described in detail. However, this is not intended to limit the present disclosure to specific embodiments, and it should be understood to include all modifications, equivalents, and substitutes included in the spirit and scope of the present disclosure. In describing the present disclosure, if it is determined that a detailed description of a related known technology may obscure the gist of the present disclosure, the detailed description thereof will be omitted.

Terms such as first, second, etc. may be used to describe various elements, but the elements should not be limited by the terms. The above terms are used only for the purpose of distinguishing one component from another.

The terms used in the present application are only used to describe specific embodiments, and are not intended to limit the present disclosure. The singular expression includes the plural expression unless the context clearly dictates otherwise. In the present application, terms such as "comprise" or "have" are intended to designate that a feature, number, step, operation, component, part, or combination thereof described in the specification exists, but one or more other features It is to be understood that this does not preclude the possibility of the presence or addition of numbers, steps, operations, components, parts, or combinations thereof.

Hereinafter, embodiments of the present disclosure will be described in detail with reference to the accompanying drawings. Here, in the description with reference to the accompanying drawings, the same or corresponding components are given the same reference numerals, and the overlapping description thereof will be omitted.

In addition, in describing various embodiments of the present disclosure, each embodiment does not have to be independently interpreted or practiced. And, it should be understood that the technical ideas described in each embodiment can be interpreted or implemented in combination in other embodiments described individually.

Hereinafter, an embodiment of the present disclosure will be described with reference to the accompanying drawings.

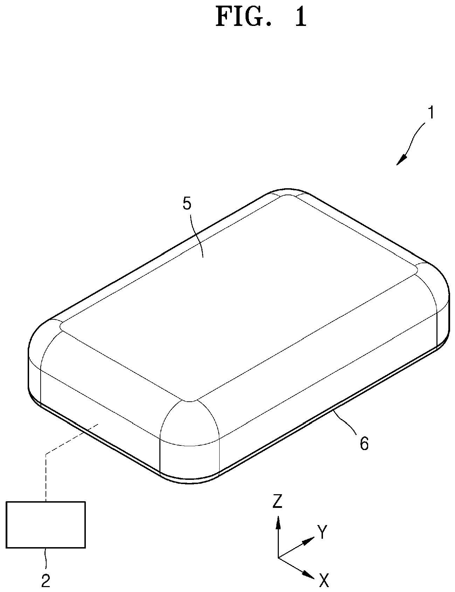

FIG. 1 is a perspective view illustrating a drug injection device 1 according to an embodiment of the present disclosure.

Referring to FIG 1, a drug injection device 1 is attached to a user's skin, etc., and may inject a drug stored therein to the user in a preset amount. The drug injection device 1 may be used for various purposes depending on the type of drug to be injected. In addition, the drug injection device 1 may be mounted on animals as well as humans to inject drugs.

The drug injection device 1 may be connected to a remote device 2 connected by wire or wirelessly. The user may use the drug injection device 1 by operating the remote device 2, and may monitor a usage state of the drug injection device 1. For example, the user may monitor an amount of drug injected from the drug injection device 1, a number of injections of the drug, an amount of drug stored in a reservoir 10, the user's bio information, etc., and may drive the drug injection device 1 based on this.

In an embodiment, the remote device 2 refers to a communication terminal capable of using an application in a wired/wireless communication environment. Here, the remote device 2 may be the user's portable terminal. In more detail, the remote device 2 may include computers (e.g., desktop, laptop, tablet, etc.), media computing platforms (e.g., cable, satellite set-top boxes, digital video recorders), any types of handheld computing devices (e.g., PDAs, email clients, etc.), hand phones, wearable devices that may be attached to or mounted on users' bodies, different kinds of computing, or any types of communication platforms, but the present disclosure is not limited thereto.

The drug injection device 1 and the remote device 2 may communicate through a communication network. In this case, the communication network refers to a communication network that provides a connection path so that the remote device 2 may transmit and receive data after accessing a service server (not illustrated). The communication network may include, for example, a wired network such as local area networks (LANs), wide area networks (WANs), metropolitan area networks (MANs), integrated service digital networks (ISDNs), or a wireless network such as wireless LANs, code division multiple access (CDMA), Bluetooth, and satellite communications, but the scope of the present disclosure is not limited thereto.

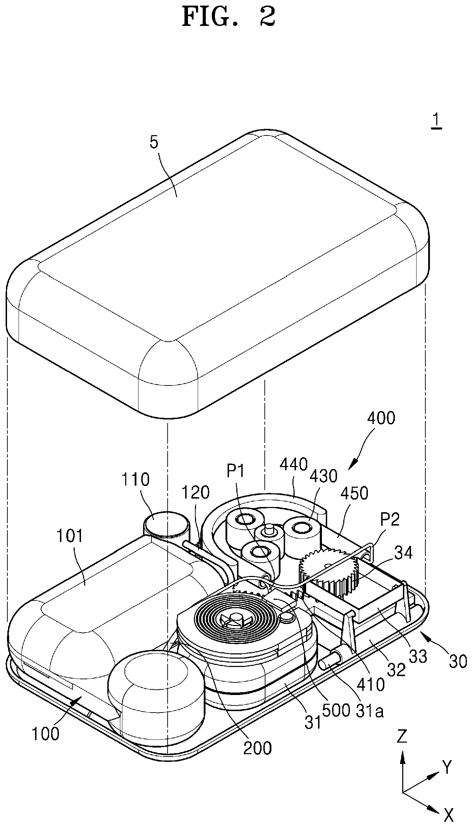

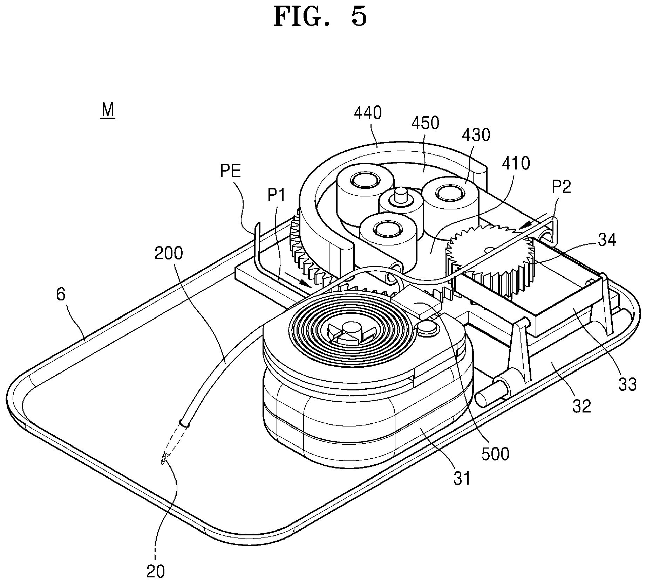

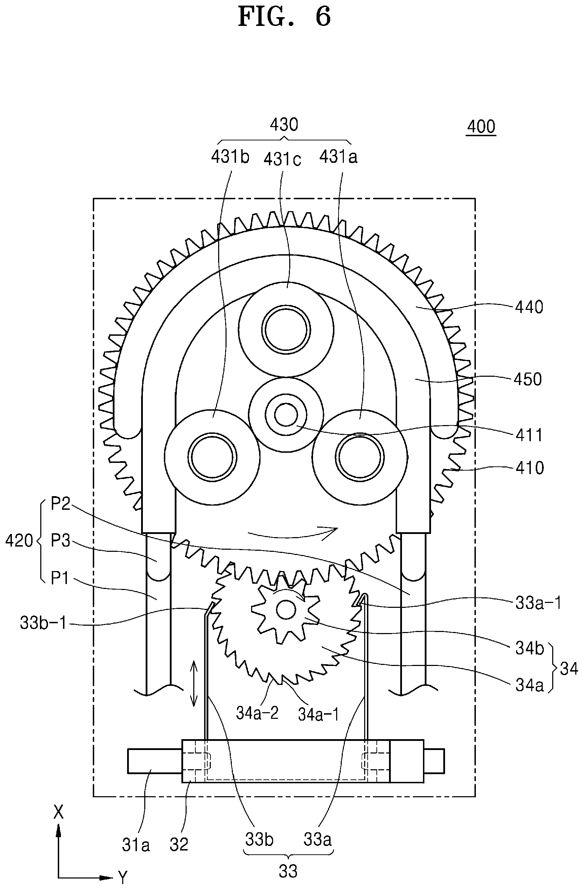

FIG. 2 is a perspective view for describing an internal arrangement of the drug injection device 1 of FIG. 1, and FIGS. 3 and 4 are views for describing a cartridge module 100 of FIG. 2. FIG. 5 is a view for describing a main body M except the cartridge module 100 in the drug injection device 1 of FIG. 2. FIG. 6 is a bottom view illustrating some parts of the main body M of FIG. 5.

Referring to FIGS. 2 to 6, the drug injection device 1 may include a base body, a cartridge module 100, a needle module 200, a driving module 30, a controller 500 and a drug discharge assembly 400.

The base body may include a housing 5 covering an outside and an attachment portion 6 attached to the user's skin, and a plurality of parts are disposed in an inner space of the housing 5.

The needle module 200 may include a needle 20 injected into the user's body to discharge the drug. The needle module 200 may be mounted on the base body, and specifically may be disposed on one side of the attachment portion 6. At this time, needle 20 may be disposed outside through the attachment portion 6 so as to be inserted into the user's body. However, the present disclosure is not limited thereto, and as another embodiment, the needle 20 may have a structure that is disposed inside the base body and protrudes to the outside as needed.

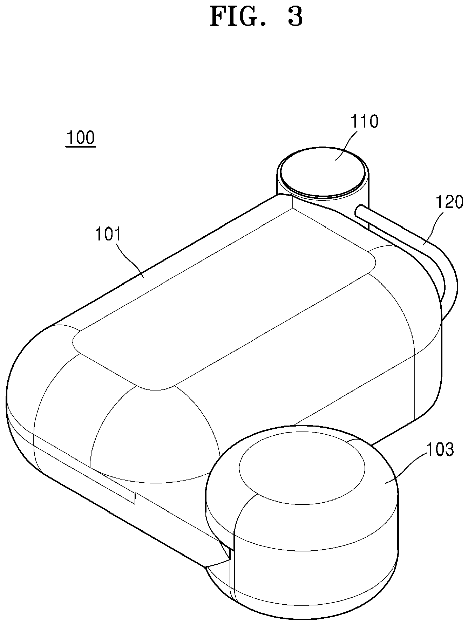

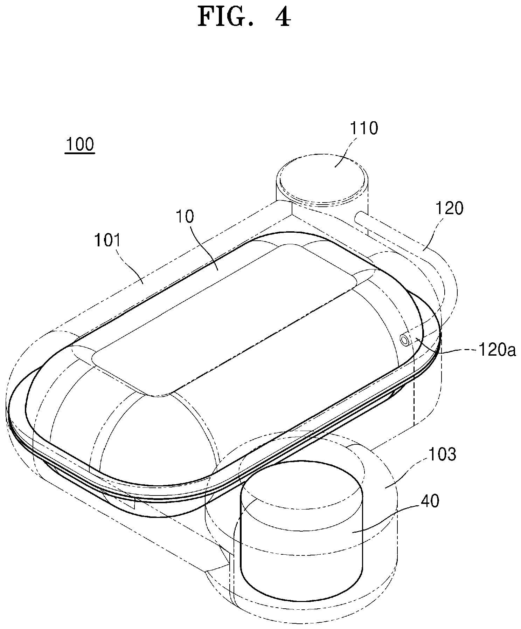

The cartridge module 100 has a reservoir 10 that stores the drug discharged to the needle 20, and may be formed attachable to and detachable from the base body. Specifically, as illustrated in FIGS. 3 and 4, the cartridge module 100 may include a case 101 that is attachable to and detachable from the attachment portion 6, and the case 101 may accommodate the reservoir 10 therein.

At this time, the cartridge module 100 may further include a connecting member 110 fluidly connecting the reservoir 10 and the drug discharge assembly 400 so that the drug in the reservoir 10 flows to the drug discharge assembly 400 when it is mounted on the base body. The connecting member 110 may be disposed on one side of the case 101, may be formed integrally with the case 101, and may be formed to have an inner space separated from the case 101.

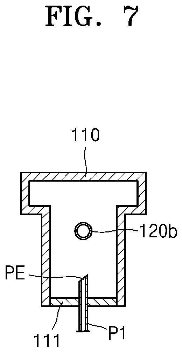

In other words, the connecting member 110 is configured to flow the drug from reservoir 10 to the drug discharge assembly 400 when the cartridge module 100 is mounted on a main body M, and may prevent the drug in the reservoir 10 from being discharged to the outside when the cartridge module 100 is not mounted on the main body M. To this end, the connecting member 110 may include a barrier rib 111 (see FIG. 7) preventing the drug from being discharged to the outside.

In addition, the cartridge module 100 may further include a battery 40 supplying power to the drug discharge assembly 400. The cartridge module 100 may further include an auxiliary case 103 accommodating the battery 40 therein. As illustrated, the auxiliary case 103 may be connected to the case 101, and more specifically, may be formed as one body. Since the user may simultaneously replace the reservoir 10 storing the drug and the battery 40 through the cartridge module 100, a convenience of the drug injection device 1 according to an embodiment of the present disclosure may be increased.

The cartridge module 100 may further include a connection pipe 120 fluidly connecting the separated connecting member 110 and reservoir 10. One end 120a of the connection pipe 120 is disposed in the inner space of the reservoir 10, and the other end 120b (see FIG. 7)

On the other hand, the drug discharge assembly 400 is disposed between the needle 20 and the reservoir 10, and dispenses the drug to the needle 20 in a preset amount. A more detailed description of the drug discharge assembly 400 will be described later.

The driving module 30 may drive the drug discharge assembly 400. The driving module 30 may be connected to the drug discharge assembly 400 to transmit driving force. The driving module 30 may include a body 31, a rotation piece 32, a drive piece 33 and a drive gear 34.

The body 31 is mounted inside the attachment portion 6 of the base body, and may rotate the rotation piece 32. The body 31 is electrically connected to the battery 40 to receive driving force, and may rotate the rotation piece 32 with respect to a rotation axis 31a.

The rotation piece 32 may be mounted on the body 31 and may rotate, and the drive piece 33 connected to the rotation piece 32 may linearly reciprocate according to the rotation of the rotation piece 32. One portion of the rotation piece 32 may be tilted by being mounted on a protrusion 31b of the body 31. Although not illustrated, the one portion of the rotation piece 32 is connected to the body 31, and the other portion may be rotated by being inserted into the rotation axis of the body 31. When the one portion of the rotation piece 32 is tilted, the other portion of the rotation piece 32 may rotate with respect to the rotation axis 31a, and the drive piece 33 may reciprocate along teeth of the drive gear 34.

As illustrated in FIG. 6, the drive piece 33 is inserted into the teeth of the drive gear 34, and may rotate the drive gear 34 in one direction during linear reciprocation. The drive piece 33 is inserted on both sides of the drive gear 34. The drive piece 33 may include a first supporter 33a inserted into one side of the drive gear 34 and a second supporter 33b inserted into the other side. The first supporter 33a may have a first end 33a-1, and the second supporter 33b may have a second end 33b-1. The first end 33a-1 and the second end 33b-1 may be bent in different directions.

Due to bending angles and directions of the first end 33a-1 and the second end 33b-1, the drive piece 33 may rotate the drive gear 34 in only one direction as described above. The bent first end 33a-1 is formed at an end portion of the first supporter 33a, and in this case, the first end 33a-1 is bent in the opposite direction of the first supporter 33a, thereby supporting teeth of a first gear 34a. Specifically, when the first supporter 33a moves downward, the first end 33a-1 moves downward while supporting a first wall 34a-1 of the teeth, so that the drive gear 34 may rotate clockwise. Also, when the first supporter 33a moves upward, the first end 33a-1 moves along a second wall 34a-2 of the teeth, so that no driving force is transmitted to the drive gear 34.

The bent second end 33b-1 is formed at an end portion of the second supporter 33b, and the second end 33b-1 is bent to have a slight slope in the second supporter 33b to move along the teeth of the drive gear 34, more specifically the first gear 34a of the drive gear 34, so that rotation in the reverse direction may be prevented. When the first end 33a-1 moves downward, the second end 33b-1 moves downward beyond the second wall 34a-2 of the teeth. In addition, when the first supporter 33a moves upward, the second end 33b-1 moves upward while supporting the first wall 34a-1 of the teeth, so that the drive gear 34 may rotate clockwise.

The drive gear 34 meshes with a rotation plate 410 of the drug discharge assembly 400. The drive gear 34 has the first gear 34a connected to the drive piece 33 and a second gear 34b meshed with the rotation plate 410. A gear ratio of the first gear 34a and the second gear 34b may be determined according to an amount of drug discharged and an injection cycle of drug.

The first supporter 33a and the second supporter 33b of the drive piece 33 may be inserted into the first gear 34a. The drive piece 33 may be supported on both sides of the first gear 34a. The first gear 34a may rotate in only one direction because of asymmetrical teeth. The first gear 34a may include the first wall 34a-1 having a large inclination and a short length, and the second wall 34a-2 having a small inclination and a long length. The drive piece 33 supports the first wall 34a-1 to transmit driving force, but moves beyond the second wall 34a-2 without supporting the second wall 34a-2.

Since the first end 33a-1 of the first supporter 33a and the second end 33b-1 of the second supporter 33b are bent in different directions, the first end 33a-1 and the second end 33b-1 alternately transmit driving force. As the drive piece 33 moves down linearly, the first end 33a-1 supports the first wall 34a-1 to rotate the drive gear 34, and the second end 33b-1 slides along the second wall 34a-2. When the drive piece 33 moves upward linearly, the first end 33a-1 slides along the second wall 34a-2, and the second end 33b-1 supports the first wall 34a-1 and rotates the drive gear 34. Thus, if the drive piece 33 is moved linearly, the drive gear 34 may continuously rotate in one direction.

The second gear 34b is formed integrally with the first gear 34a. When the driving force is transmitted to the first gear 34a, the second gear 34b may be rotated and rotate the rotation plate 410.

The controller 500 may control the operation of drug injection device 1. The controller 500 may control the operation of the driving module 30 to control a discharge cycle of drug, a discharge amount and a discharge speed.

Hereinafter, the drug discharge assembly 400 will be described in detail, and a method in which the cartridge module 100 is connected to the drug discharge assembly 400 will be described.

FIG. 7 is a view illustrating a state in which a connecting member of the cartridge module and a tube of a drug discharge assembly are connected.

Referring to FIGS. 5, 6 and 7, the drug discharge assembly 400 may include the rotation plate 410, a tube 420, a pressing portion 430, a guide portion 440 and a cover 450. The drug discharge assembly 400 may compress the tube 420 to dispense the drug moving through the tube 420 in a preset amount.

The rotation plate 410 may rotate in one direction. The rotation plate 410 meshes with the drive gear 34 and rotates in one direction by the drive gear 34 rotating in only one direction. In FIG. 6, the drive gear 34 rotates only in the clockwise direction, so the rotation plate 410 may rotate only in the counterclockwise direction.

The rotation plate 410 may be inserted into a support shaft (not illustrated) fixed to the base body and rotate around the support shaft (not illustrated). The rotation plate 410 may include a rib 411 protruding to surround the support shaft (not illustrated) and a plurality of fixed protrusions 312 supporting the pressing portion 430.

In an alternative embodiment, bearings may be installed between the support shaft (not illustrated) and the rotation plate 410 and between the fixed protrusion 312 and the pressing portion 430, through which the pressing portion 430 may rotate.

Tube 420 is installed between the reservoir 10 and the needle 20, and may be installed to pass through the rotation plate 410. A portion of the tube 420 may extend in a circumferential direction of the rotation plate 410. The tube 420 is formed of a flexible material so as to be compressed when the pressing portion 430 is in close contact.

The tube 420 may include an inlet end P1 introduced from the reservoir 10, and an outlet end P2 discharged to the needle 20. In addition, a curved portion P3 is formed between the inlet end P1 and the outlet end P2, and the curved portion P3 may extend along the circumferential direction of the rotation plate 410. Since the curved portion P3 is disposed to be spaced apart from a surface of the rotation plate 410, even when the rotation plate 410 rotates, it is fixed at a preset position.

The curved portion P3 branches from the inlet end P1 to the outlet end P2, and the branched curved portion P3 extends in parallel to each other in the circumferential direction. The curved portion P3 may extend to both sides of the rotation plate 410. At this time, although not illustrated, a first curved tube may be disposed in the circumferential direction along one surface of the rotation plate 410, and a second curved tube may be disposed in the circumferential direction along the other surface of the rotation plate 410.

Since the tube 420 extends on both sides of the rotation plate 410, it may continuously and precisely dispense the drug. If the tube is extended with only one tube, a rotation speed of the rotation plate must be increased to discharge a large amount of drug because a period for precisely controlling and dispensing the drug is determined by a rotation period of the rotation plate 410. However, if the rotation speed of the rotation plate is excessively increased, stability and durability are deteriorated due to overload and heat of the driving unit.

Since the tube 420 according to an embodiment of the present disclosure extends on both sides of the rotation plate 410, the discharge cycle of drug may be shortened, thereby reducing the overload of the driving module 30 and improving durability and stability. Also, since the first curved tube and the second curved tube have different discharge cycles, the amount of drug may be precisely controlled.

On the other hand, the tube 420 according to an embodiment of the present disclosure may be fluidly connected to the cartridge module 100 through an insertion portion PE of the inlet end P1. As illustrated in FIG. 5, the inlet end P1 of the tube 420 may include the insertion portion PE extending vertically from a bottom surface of the attachment portion 6 to engage the cartridge module 100.

At this time, the insertion portion PE may be formed to be inserted into the inside of the connecting member 110 of the cartridge module 100. For example, the insertion portion PE may be formed in a pointed shape as illustrated in FIG. 7. The connecting member 110 may have the barrier rib 111 penetrable by the insertion portion PE. When the user applies pressure after matching the barrier rib 111 of the connecting member 110 to the position of the insertion portion PE in order to mount the cartridge module 100 to the main body M, the insertion portion PE may pass through the barrier rib 111, and may be inserted into the inside of the connecting member 110.

Meanwhile, the pressing portion 430 may be mounted on the rotation plate 410 to press the tube 420 when the rotation plate 410 rotates. The pressing portion 430 may include a plurality of rollers spaced apart from each other at predetermined intervals along the circumferential direction of the rotation plate 410.

The pressing portion 430 may be inserted into the fixed protrusion 312 of the rotation plate 410 to rotate around the fixed protrusion 312. That is, the pressing portion 430 may revolve around the support shaft (not illustrated) by rotation of the rotation plate 410 while rotating about the fixed protrusion 312 of the rotation plate 410. Since the pressing portion 430 rotates, the friction of the tube 420 may be reduced and the tube 420 may be compressed gently. Since the pressing portion 430 revolves, the pressing portion 430 may periodically discharge the drug in a preset amount.

The pressing portion 430 may be mounted on both sides of the rotation plate 410. A first roller is mounted on one surface of the rotation plate 410 and may apply the first curved tube. A second roller is mounted on the other surface of the rotation plate 410 and may apply the second curved tube. The first roller and the second roller may be disposed to be shifted from each other, so that the tube 420 may be applied at different cycles.

Since the first roller and the second roller have different rotation cycles, the drug is alternately discharged from the first curved tube and the second curved tube. As a result, the drug discharge assembly 400 discharges the drug in a short cycle, so that the drug may be injected quickly and accurately. Hereinafter, the first roller mounted on one surface of the rotation plate 410 will be mainly described.

The first roller may include a first-a roller 431a, a first-b roller 431b, and a first-c roller 431c which are disposed at an interval of 120 degrees from each other. However, the number of first rollers is not limited, and may be set variously according to the discharge cycle of the drug and discharge amount.

The guide portion 440 extends along the circumferential direction of the rotation plate 410, and may support the tube 420. The guide portion 440 extends along the curved portion P3 and supports one side of the tube 420, so that the tube 420 may be compressed when the first roller rotates.

Although the drawing illustrates an embodiment in which the guide portion 440 is disposed on an outside of the tube 420 and the pressing portion 430 is disposed on an inside of the tube 420, it is not limited thereto, and in another embodiment, the guide portion may be disposed on the inside of the tube, and the pressing portion may be disposed on the outside of the tube.

Since the guide portion 440 is fixed to the base body, the position of the tube 420 may be aligned. The guide portion 440 is spaced apart from the rotation plate 410 by a predetermined interval, so that the position may be fixed even when the rotation plate 410 rotates.

The cover 450 is installed to surround the tube 420 pressed by the pressing portion 430. The cover 450 is installed to cover the curved portion P3, and may be formed of a flexible material. The cover 450 may prevent the curved portion P3 from being damaged by the pressing portion 430. Since the cover 450 is in contact with the pressing portion 430 on the outer circumferential surface of the curved portion P3, the force transmitted from the pressing portion 430 may be dispersed, thereby preventing the curved portion P3 from being damaged by the pressing portion 430.

The curved portion P3 of tube 420 has a curvature and extends in the circumferential direction, so it may be easily damaged by external force. Since the cover 450 covers the tube 420, the durability of the tube 420 may be increased.

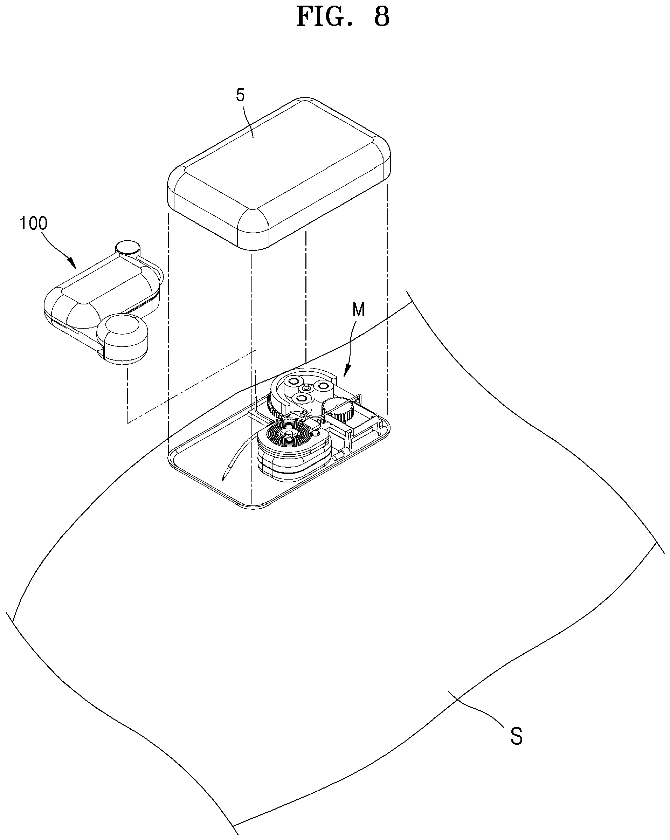

FIG. 8 is a view for describing a method of using a drug injection device 1 according to an embodiment of the present disclosure.

Referring to FIG. 8, when the user attaches the attachment portion of the drug injection device 1 to a skin S of a drug injection position, the needle 20 of the drug injection device 1 may be inserted into the skin S of the user. At this time, the drug injection device 1 includes the cartridge module 100, so that the drug in the reservoir 10 may be discharged through the needle 20.

On the other hand, since the reservoir 10 accommodates only a certain amount of drug, conventionally, after drug injection for a certain period of time, for example, after drug injection for 3.5 days, the entire drug injection device 1 had to be replaced. In this case, the needle 20 had to be inserted into the user's skin S again.

In the drug injection device 1 according to an embodiment of the present disclosure, only the cartridge module 100 may be replaced while the main body M on which the needle 20 is mounted remains, so that the mounting time of the main body M of the drug injection device 1 may be extended, and thus, not only the user's inconvenience but also the pain caused by the needle 20 replacement may be reduced.

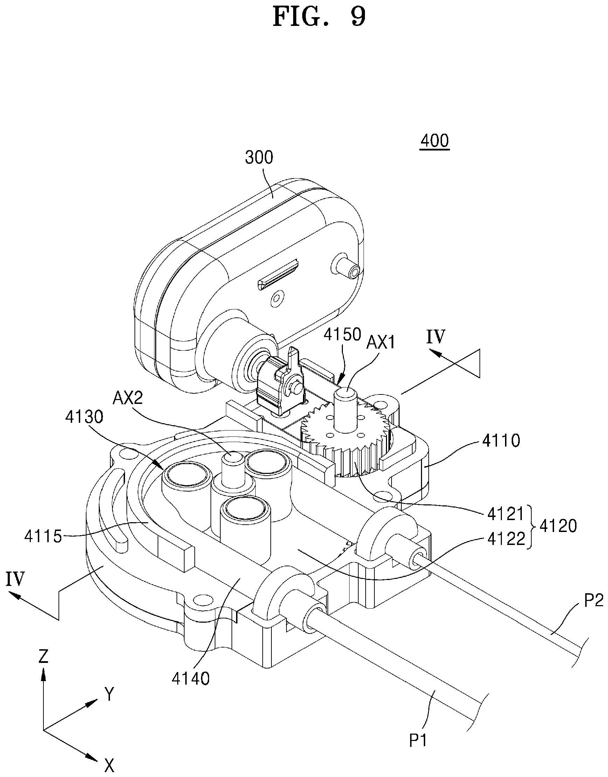

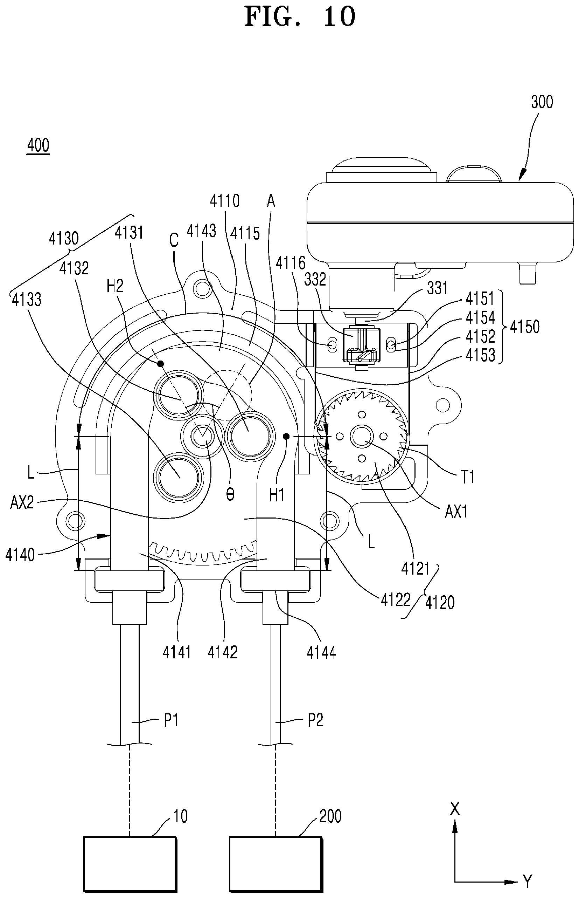

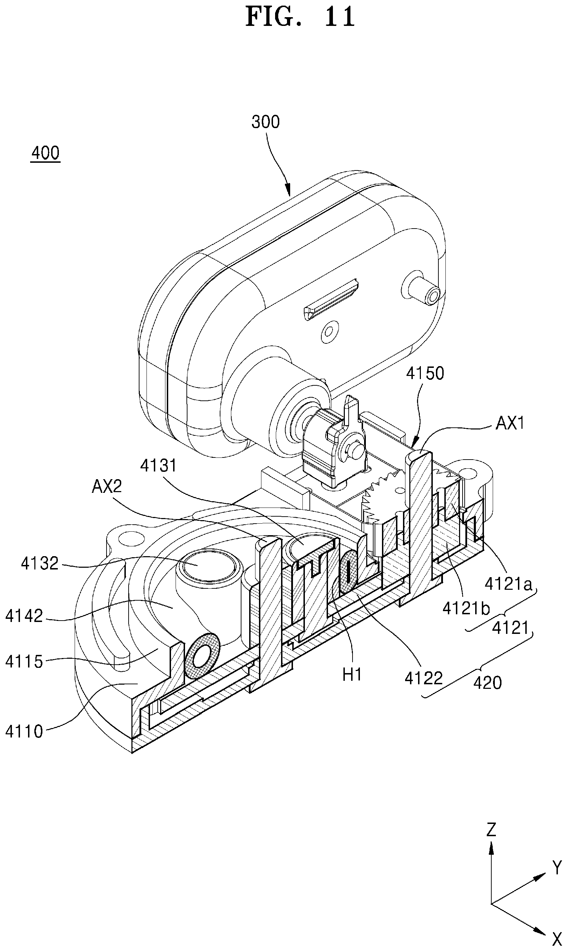

FIG. 9 is a perspective view illustrating a drug discharge assembly 400 according to another embodiment of the present disclosure, FIG. 10 is a plan view illustrating the drug discharge assembly 400 of FIG. 9, and FIG. 11 is a cross-sectional view taken along IV-IV of FIG. 9.

A drug injection device 1 according to another embodiment may include a reservoir 10, a needle module 200, a driving module 300, and a drug discharge assembly 400.

The reservoir 10 stores a drug to be injected, and is connected to the drug discharge assembly 400. The reservoir 10 may be mounted inside a housing 5. In another embodiment, the reservoir 10 may be installed outside the housing 5 as well.

The needle module 200 is disposed on one side of housing 5. Since the needle module 200 is inserted into the user's skin, the drug to be discharged may be injected into the user. The drug discharge assembly 400 is installed between the needle module 200 and the reservoir 10, so that the drug may be dispensed at a preset amount or at a preset cycle and discharged to the needle module 200.

The driving module 300 is disposed in an inner space of the housing 5, and may transmit a driving force to the drug discharge assembly 400. In one embodiment, each of the driving module 300 and the drug discharge assembly 400 may be arranged as separate components. In another embodiment, the driving module 300 may be composed of a component constituting the drug discharge assembly 400.

Referring to FIGS. 9 to 11, the driving module 300 may include a drive shaft 331 linearly reciprocating in one direction. When the driving module 300 is driven, the drive shaft 331 may repeatedly advance and retract in one direction, that is, in a longitudinal direction. A joint 332 may be installed at one side of the drive shaft 331. The joint 332 is connected to a drive piece 4150 so that the linear reciprocating motion of the drive shaft 331 may cause the drive piece 4150 to linearly reciprocate.

All kinds of devices with drug suction power and drug discharge power by electricity may be used as the driving module 30. For example, all kinds of pumps such as mechanical displacement type micropumps and electromagnetic motion type micropumps may be used. The mechanical displacement micropump is a pump that uses a motion of a solid or fluid, such as a gear or diagram, to generate a pressure difference to induce a flow of fluid, and includes a diaphragm displacement pump, a fluid displacement pump, a rotary pump, and the like. The electromagnetic motion type micropump is a pump that directly uses electrical or magnetic energy to move a fluid, and includes an electro hydrodynamic pump (EHD), an electro osmotic pump, a magneto hydrodynamic pump, an electro wetting pump, and the like.

The drug discharge assembly 400 includes a base 4110, a rotation unit 4120, a pressing unit 4130, a tube 4140 and the drive piece 4150, and the driving module 300 may be connected to the drive piece 4150.

The base 4110 supports the drug discharge assembly 400 and may form an exterior. At least one of the rotation unit 4120, the pressing unit 4130, the tube 4140 and the drive piece 4150 may be installed and supported on the base 4110. The base 4110 is mounted on the housing 5, so that the drug discharge assembly 400 may be installed in the inner space of the drug injection device 1.

The base 4110 may include a guide portion 4115. The guide portion 4115 may extend along a circumferential direction of a second rotation member 4122 and support the tube 4140. One portion of the guide portion 4115 protrudes from one surface of the base 4110, and may extend along a curve section C of the tube 4140. In addition, the other portion of the guide portion 4115 may extend to a straight section L of the tube 4140.

The guide portion 4115 may support the force applied by the pressing unit 4130 to guide the movement of the drug. When the pressing unit 4130 applies the tube 4140, the guide portion 4115 supports the tube 4140 on the opposite side of the pressing unit 4130. When the pressing unit 4130 applies the tube 4140 at the curve section C, the tube 4140 may be compressed so that an internal cross-sectional area through which the drug flows is zero at pressing points H1 and H2 where the tube 4140 and the pressing portion contact. At this time, when the pressing unit 4130 rotates, the drug in tube 4140 also moves. In one embodiment, the guide portion 4115 may be disposed on an outside of the curve section C, and a region where the pressing unit 4130 applies the tube 4140 may be disposed on an inside of the curve section C.

Although the drawing illustrates an embodiment in which the guide portion 4115 is disposed on the outside of the tube 4140 and the pressing unit 4130 is disposed on the inside of the tube 4140, it is not limited thereto, and in another embodiment, the guide portion may be disposed on the inside of the tube, and the pressing unit may be disposed on the outside of the tube.

The guide portion 4115 may protrude from the base 4110 to align the position of the tube 4140. Since the guide portion 4115 is formed along the curve section C, and the tube 4140 is extended along the guide portion 4115, the guide portion 4115 may form and align the curve section C.

The base 4110 may include a guide protrusion 4116. The guide protrusion 4116 protrudes from one surface of the base 4110 and may be inserted into a guide hole 4154 of the drive piece 4150. In one embodiment, the guide protrusion 4116 is provided as a pair and is respectively inserted into a pair of guide holes 4154 to guide the movement of the drive piece 4150.

The base 4110 may have a guide wall 4117. The guide wall 4117 protrudes from one surface of the base 4110, and may guide the movement of the drive piece 4150. The guide wall 4117 is disposed adjacent to the guide protrusion 4116 and may support a first arm 4152 and a second arm 4153 of the drive piece 4150.

By means of the guide protrusion 4116 and the guide wall 4117, the drive piece 4150 may be guided in linear reciprocating motion. The guide protrusion 4116 is inserted into the guide hole 4154, and each of the first arm 4152 and the second arm 4153 are inserted into the guide wall 4117 extending in a moving direction, so that the drive piece 4150 may move along a preset trajectory during linear motion.

The rotation unit 4120 is mounted on one side of the base 4110 and may rotate by receiving the driving force from the driving module 300. The rotation unit 4120 is in contact with an end of the drive piece 4150, and may rotate in one direction according to the linear reciprocating motion of the drive piece 4150. The rotation unit 4120 may be defined as a configuration in which at least some components are rotated by receiving the driving force from the driving module 300 to rotate a pressing portion 4130.

In an embodiment, a plurality of members of the rotation unit 4120 may be drivably connected. The rotation unit 4120 may include a first rotation member 4121 and the second rotation member 4122.

The first rotation member 4121 may contact the end of the drive piece 4150 and rotate according to the linear reciprocating motion of the drive piece 4150. The second rotation member 4122 may be connected to the first rotation member 4121 to rotate according to the rotation of the first rotation member 4121.

The first rotation member 4121 may include a first rotation stage 4121a disposed to contact at least one of the first arm 4152 and the second arm 4153 of the drive piece 4150. The first rotation stage 4121a may receive the driving force from the drive piece 4150 to rotate about a first axis AX1.

The first rotation stage 4121a may include a plurality of first teeth T1. The first teeth T1 are disposed in a circumferential direction of the first rotation stage 4121a, and may include a first surface S1 and a second surface S2 having different lengths. This will be described in detail below.

The first rotation member 4121 may include a second rotation stage 4121b disposed on the opposite side of the first rotation stage 4121a. The second rotation stage 4121b is integrally formed with the first rotation stage 4121a, and may rotate about the first axis AX1 together with the first rotation stage 4121a receiving the driving force.

The second rotation member 4122 has a plate shape and may rotate about a second axis AX2. The second rotation member 4122 is connected to the second rotation stage 4121b. A second teeth of the second rotation stage 4121b and a third teeth of the second rotation member 4122 are connected to each other, so that the second rotation member 4122 may be rotated by the rotation of the second rotation stage 4121b.

A rotation angle of the second rotation member 4122 may be determined by a number of movements of the drive shaft 331, a number of first teeth, a number of second teeth, and a number of third teeth. In detail, a rotation angle of the first rotation member 4121 according to the driving of the drive shaft 331 is set by the number of first teeth. Also, the rotation angle of the second rotation member 4122 according to the driving of the drive shaft 331 may be determined by a ratio of the number of first teeth to the number of second teeth, and a ratio of the number of second teeth to the number of third teeth. Accordingly, by adjusting the number of first teeth, second teeth, and third teeth, the discharge amount of the drug according to one-time driving of the driving module 300 may be adjusted.

In another embodiment, the rotation unit may be provided as a single rotation member, and may receive a driving force directly from the driving module. The drive piece may directly rotate the rotation member by pressing the rotation member on which the pressing portion is installed. When the drive shaft of the driving module moves linearly, the drive piece moves together linearly to rotate the rotation member. The pressing portion installed on the rotation member applies the tube to discharge the drug.

The pressing unit 4130 is mounted on the rotation unit 4120, and may rotate together with the rotation unit 4120. The pressing unit 4130 may apply the tube 4140 while rotating about the second axis AX2. The pressing unit 4130 may apply the tube 4140 when in contact with the curve section C of the tube 4140 such that the tube 4140 is compressed at a contact point.

The pressing unit 4130 may include a plurality of rollers. When the drug discharge assembly 400 is driven, at least one pressing portion may be disposed on the curve section C. More preferably, when the pressing unit 4130 rotates, at least nine pressing portions may form the pressing points H1 and H2 in the curve section C.

As described above, at the contact point between the pressing portion of the pressing unit 4130 and the tube 4140, the tube is compressed by the pressing portion, so that the internal cross-sectional area of the tube 4140 is formed to be zero. Since at least nine pressing points H1 and H2 are formed in the curve section C, a preset amount of drug may be discharged according to a rotation angle of the second rotation member 4122. In detail, since the amount of drug does not change between two pressing points H1 and H2 formed by the pressing portion in the curve section C, the drug is primarily distributed in a preset amount. Thereafter, the drug primarily distributed may be secondarily discharged in a preset amount by rotation of the second rotation member 4122.

In the pressing unit 4130, the number of pressing portions may be set according to the length of the curve section C. The pressing portion may be determined by a central angle of the curve section. The number of pressing portions may be set according to the following equation.

### [Equation]

N > 360° / central angle (degree) of curve section

N is the number of pressing portions included in the pressing unit 4130, and the central angle of the curve section is defined as the central angle of the curve section C about the second axis AX2.

In an embodiment, as illustrated in FIG. 10, if the central angle of the curve section C is 180 degrees, the number of pressing portions forming the pressing unit 4130 may be set to three or more. When the central angle of the curve section C is formed by 180 degrees and three or more pressing portions are provided, at least nine pressing points H1 and H2 may always be formed in the curve section C. Therefore, in order to distribute the drug primarily by the pressing points H1 and H2 in the curve section C, the number of pressing portions may be set according to the above equation.

In an embodiment, the pressing unit 4130 may include a first pressing portion 4131, a second pressing portion 4132, and a third pressing portion 4133. The equally spaced first pressing portion 4131, second pressing portion 4132 and third pressing portion 4133 form pressing points with tube 4140, and when the second rotation member 4122 is rotated, the pressing points H1 and H2 move along the curve section C.

In an embodiment, the pressing unit 4130 may include a contact cover (not illustrated) having a predetermined hardness on an outside of each of the pressing portions. The contact cover (not illustrated) may be formed of a material having a predetermined cushion, gently press the flexible tube 4140, and completely compress the tube 4140.

The tube 4140 is disposed adjacent to the rotation unit 4120, and may include the curve section of which at least a portion extends in the circumferential direction. The tube 4140 is formed of a flexible material and may be compressed by the pressing portion of the pressing unit 4130.

The tube 4140 is installed between the reservoir 10 and the needle 20, and may be installed to pass through the rotation unit 4120. A portion of the tube 4140 may extend in the circumferential direction of the second rotation member 4122.

The tube 4140 may include an inlet end 4141 connected to the reservoir 10 through a first pipe P1, and an outlet end 4142 connected to the needle module 200 through a second pipe P2. Also, a curved portion 4143 is formed between the inlet end 4141 and the outlet end 4142, and the curved portion 4143 may extend in the circumferential direction of the second rotation member 4122. The inlet end 4141 and the outlet end 4142 may form a straight section L, and the curved portion 4143 may form a curve section C.

In one embodiment, referring to FIG. 10, the curved portion 4143 may have a central angle of 180 degrees with respect to the second axis AX2. However, the central angle of the curved portion 4143 is not limited thereto, and may be variously set according to the discharge amount of the drug, the number of pressing portions, and the like.

In the curve section C, the tube 4140 is compressed by the pressing portion, so that the internal cross-sectional area of the tube 4140 at the pressing points H1 and H2 may be formed to zero. On the other hand, in the straight section L, while the contact or pressure between the pressing portion and the tube 4140 is released, the drug may be discharged to the outlet end 4142 according to the movement of the pressing portion.

The tube 4140 may have a fixing block 4144 mounted on the base 4110. The fixing block 4144 is arranged at the inlet end 4141 and the outlet end 4142, respectively, and the fixing block 4144 is mounted on the base 4110, so that the position of the tube 4140 may be fixed.

The drive piece 4150 may be disposed between the driving module 300 and the rotation unit 4120 to transmit the driving force generated by the driving module 300 to the rotation unit 4120. The drive piece 4150 is connected to the drive shaft 331 so as to reciprocate linearly according to the movement of the drive shaft 331.

The drive piece 4150 may include a driving body 4151, a first arm 4152, a second arm 4153 and a guide hole 4154.

The driving body 4151 may be connected to the drive shaft 331. Specifically, a joint 332 is disposed between the driving body 4151 and the drive shaft 331, so that the driving body 4151 may linearly reciprocate when the drive shaft 331 linearly reciprocates.

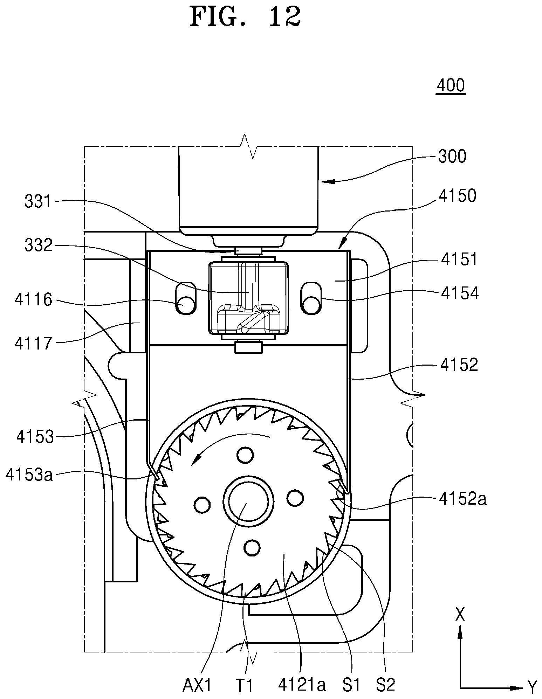

The first arm 4152 may extend from one side of the driving body 4151. A first bent end 4152a bent at a predetermined angle with respect to a longitudinal direction may be formed at an end of the first arm 4152. Referring to FIG. 12, the first bent end 4152a is bent toward the driving module 300, and may have a bending angle smaller than 90 degrees in the longitudinal direction of the first arm 4152.

The second arm 4153 may extend from the other side of the driving body 4151 and may be arranged parallel to the first arm 4152. A second bent end 4153a bent in a direction different from that of the first bent end 4152a may be formed at an end of the second arm 4153. Referring to FIG. 12, the second bent end 4153a extends in a direction away from the driving module 300, and may have a bending angle exceeding 90 degrees in a longitudinal direction of the second arm 4153.

Since the first arm 4152 and the second arm 4153 may have a predetermined elasticity, the first bent end 4152a and the second bent end 4153a may apply a first surface S1 of the first teeth T1 when the drive piece 4150 is linearly reciprocated in a front-rear direction, or may move along a second surface S2 of the first teeth T1.

The guide hole 4154 may be installed in the driving body 4151 and may have a larger opening area than the cross-section of the guide protrusion 4116. The guide hole 4154 has an elongated shape so that the drive piece 4150 may move along the guide protrusion 4116 in linear reciprocating motion.

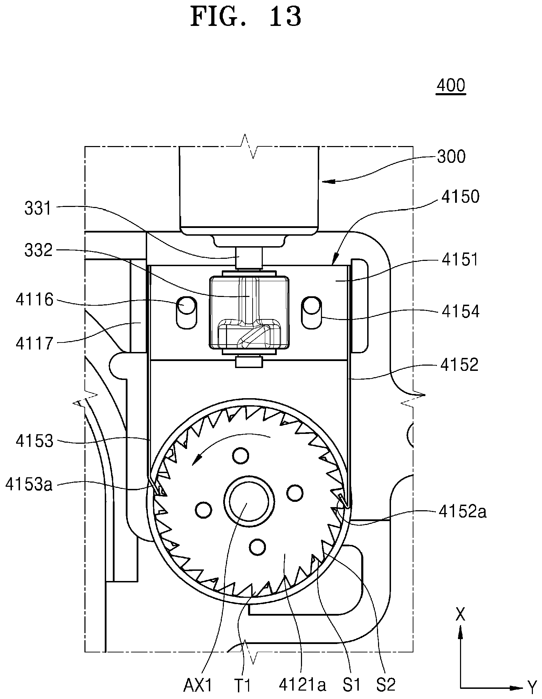

FIGS. 12 and 13 are diagrams illustrating an operation of the drug discharge assembly 400.

Referring to FIGS. 12 and 13, in the drug discharge assembly 400, the drive shaft 331 linearly reciprocates in the front-rear direction to discharge the drug in a preset amount.

Hereinafter, moving forward is defined as the movement of the drive shaft 331 toward the first axis AX1, and moving backward is defined as the movement of the drive shaft 331 toward the driving module 300.

The first teeth T1 may include a first surface S1 and a second surface S2 having different lengths in an inclined direction. The length of the first surface S1 is smaller than the length of the second surface S2. That is, the second surface S2 may have a gentler slope than the first surface S1.

The first surface S1 is applied by the first bent end 4152a or the second bent end 4153a, so that the first rotation member 4121 may rotate in one direction. On the other hand, in the second surface S2, the first bent end 4152a or the second bent end 4153a may move beyond the second surface S2. Since the drive piece 4150 transmits the driving force only to the first surface S1, the first rotation member 4121 may rotate in only one direction.

As illustrated in FIG. 12, when the drive shaft 331 moves backward, the first bent end 4152a moves backward while in contact with the first surface S1 so that the first rotation member 4121 may rotate by one tooth angle. At this time, the second bent end 4153a moves along the second surface S2.

As illustrated in FIG. 13, when the drive shaft 331 moves forward, the second bent end 4153a moves forward while in contact with the first surface S1 so that the first rotation member 4121 may rotate by one tooth angle. At this time, the first bent end 4152a moves along the second surface S2.

Since the first bent end 4152a and the second bent end 4153a are bent in different directions, and the first surface S1 and the second surface S2 in the first teeth T1 have different inclinations, the first rotation member 4121 may move in one direction by the linear reciprocating motion of the drive piece 4150.

The rotation unit 4120 is always drivably connected to any one of the first bent end 4152a and the second bent end 4153a. The first bent end 4152a and the second bent end 4153a alternately apply the rotation unit 4120 during the linear reciprocating motion of the drive piece 4150. When the drive piece 4150 linearly reciprocates in the front-rear direction, the first bent end 4152a or the second bent end 4153a always applies the first surface S1 of the first teeth T1, so the first rotation member 4121 may rotate continuously.

When the first rotation member 4121 rotates, the second rotation member 4122 may also rotate. According to the rotation of the second rotation member 4122, the pressing unit 4130 applies the tube 4140, so that the drug may be primarily distributed. Since the cross-sectional area of the tube 4140 becomes zero at the point where the pressing portion of the pressing unit 4130 and the tube 4140 come into contact and press, the drug is primarily dispensed in the space between the pair of pressing points H1 and H2.

When the second rotation member 4122 rotates, the pressing unit 4130 also moves together so as to move the primarily dispensed drug to the needle module 200 to finally discharge the drug in a preset amount. According to the driving mechanism as described above, the drug is dispensed twice while passing through the drug discharge assembly 400, so that a preset amount of drug may be discharged into the needle module 200.

The pressing unit 4130 may control the amount of drug that is primarily dispensed by adjusting the number of pressing portions. For example, when the number of pressing portions is increased, the amount of the drug to be primarily dispensed may be set to be small, and the drug to be finally discharged may be accurately dosed in a preset amount.

The drug discharge assembly 400 may control the amount of drug discharged by a rotation angle of the rotation unit 4120 and a radial length of the rotation unit 4120. The rotation angle of the rotation unit 4120 and the radial length of the rotation unit 4120 set a flow distance of drug in the curve section C. Since the rotation angle of the second rotation member 4122 and a radial distance of the second rotation member 4122 based on the second axis AX2 set the flow distance of drug, the drug may be discharged in a preset amount.

Referring to FIG. 10, when the second rotation member 4122 rotates by θ, the second pressing portion 4132 also rotates and moves to a position A. By adjusting the rotation angle of the second rotation member 4122, it is possible to precisely control the amount of drug discharged by moving an applying point of the second pressing portion 4132.

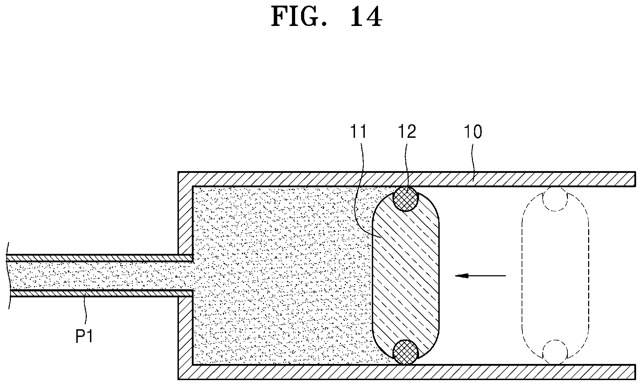

FIG. 14 is a view illustrating a reservoir 10 connected to the drug discharge assembly 400 of FIG. 9.

Referring to FIGS. 10 and 14, since the drug is moved from the reservoir 10 to the needle module 200 by driving the drug discharge assembly 400, an additional driving source for discharging the drug to the reservoir 10 is unnecessary.

The reservoir 10 has a space to store the drug inside. One side of the reservoir 10 is connected to the tube 4140 by the first pipe P1, and a plunger 11 is disposed on the other side. In plunger 11, a sealing member 12 is disposed on a portion in contact with an inner wall of the reservoir 10, thereby preventing drug leakage through the plunger 11.

Conventionally, in order to discharge the drug from the reservoir to the needle assembly, the driving module accurately pushed the plunger, and the drug was discharged into the needle assembly by the movement of the plunger. However, there is a limit to implementing a mechanism for precisely moving the plunger in order to discharge the drug in a preset manner.

The drug discharge assembly 400 may discharge a preset amount of the drug stored in the reservoir 10 to the needle module 200 without providing the additional driving source to the reservoir 10.

As the pressing portions of the driving module 300 rotate while compressing the tube 4140, the drug present in the tube 4140 moves to the needle module 200. When the drug present in the tube 4140 is discharged into the needle module 200, the drug stored in reservoir 10 flows into an empty space of the tube 4140. Therefore, even if the additional driving source is not provided to the reservoir 10, the drug in the reservoir 10 moves to the tube 4140 along the first pipe P1, and the drug dispensed in a preset amount from the drug discharge assembly 400 moves to the second pipe P2 to be discharged to the needle module 200.

The drug discharge assembly 400 and the drug injection device 1 according to an embodiment of the present disclosure may inject a drug into a subject in a constant manner. When the drive shaft 331 of the driving module 300 linearly reciprocates, the rotation angle of the rotation unit 4120 is adjusted, and the amount of drug moving along the curve section C is controlled, so that the drug may be discharged in a preset amount into the needle module 200.

The drug discharge assembly 400 and the drug injection device 1 according to an embodiment of the present disclosure dispense the drug multiple times, so that a small amount of the drug may be accurately discharged. Since the pressing portion of the pressing unit 4130 compresses the tube 4140, a preset amount of drug is primarily dispensed between the pair of pressing points H1 and H2. Since the drug primarily dispensed is secondarily dispensed while moving the curve section C, the drug discharge assembly 400 accurately dispenses a small amount of drug, and the drug injection device 1 may inject the drug into the subject in a preset amount.

The drug discharge assembly 400 and the drug injection device 1 according to an embodiment of the present disclosure may accurately dispense the drug with a simple driving mechanism. Even if an additional driving source is not provided to the reservoir 10, the drug may move from the reservoir 10 to the needle module 200 by rotating the pressing unit 4130. Since the drug discharge assembly 400 functions as a driving source to move the drug while performing the function of dispensing the drug, the structure of the drug injection device 1 may be formed simply and compactly.

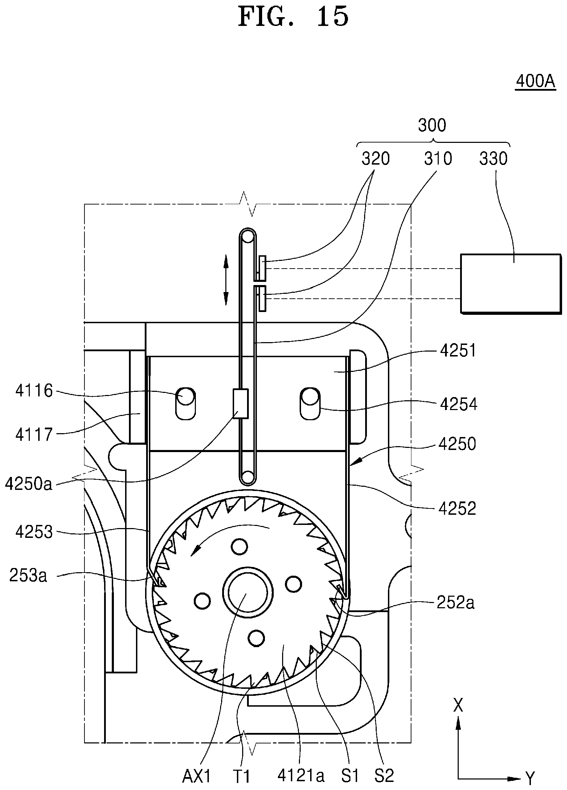

FIG. 15 is a diagram illustrating a drug discharge assembly 400A according to another embodiment of the present disclosure.

Referring to FIG. 15, in a drug discharge assembly 400A, a driving module 300 may linearly move a drive piece 4250.

The drive piece 4250 is similar to the drive piece 4150 of the embodiment described above, and the drive piece 4250 may include a body 4251, a first arm 4252, a second arm 4253 and a guide hole 4254. In addition, a second bent end 252a may be provided at an end of the first arm 4252, and a second bent end 253a bent in a direction opposite to the first bent end may be provided at an end of the second arm 4253.

The drive piece 4250 may include a connector 4250a connected to the driving module 300. The connector 4250a is connected to a wire 310 of the driving module 300 and may linearly move in one direction according to the movement of the wire 310. The driving module 300 may transmit a driving force to the drive piece 4250 to linearly reciprocate the drive piece 4250. The driving module 300 may include the wire 310, a connection terminal 320 and a control unit 330.

The wire 310 is connected to the drive piece 4250, and may move the drive piece 4250 according to the contraction and extension of the wire 310. The wire 310 may contract or extend in length according to a control signal applied from the control unit 330.

In one embodiment, the wire 310 may be formed of a shape-memory alloy (SMA). The wire 310 may be formed of a well-known shape-memory material and is not limited to a specific material. For example, it may be formed of an alloy of nickel and titanium.

The connection terminal 320 is disposed at both ends of the wire 310, and receives an electrical signal from the control unit 330. The connection terminal 320 may be formed of a material having electrical conductivity.

The control unit 330 may generate and control a current signal. The control unit 330 may control the current applied to the connection terminal 320. The control unit 330 may be connected to a battery (not illustrated) to control whether current is applied from the battery to the connection terminal 320.

For example, the control unit 330 may alternately apply an electrical signal to the connection terminal 320, whereby the wire 310 may linearly reciprocate along one direction. That is, when a current is applied to only one of the connection terminals 320, the drive piece 4250 moves forward by the contraction or extension of one end of the wire 310. In addition, when a current is applied only to the other one of the connection terminals 320, the drive piece 4250 moves backward to its original position due to the contraction or extension of the other end of the wire 310. As the control unit 330 controls the current signal applied to the connection terminal 320, the wire 310 contracts or extends, so that the drive piece may reciprocate in the front-rear direction.

As such, the present disclosure has been described with reference to the embodiments shown in the drawings, which are merely exemplary, and those of ordinary skill in the art will understand that various modifications and equivalent other embodiments are possible therefrom. Therefore, the true technical protection scope of the present disclosure should be determined by the technical spirit of the appended claims.

### INDUSTRIAL APPLICABILITY

According to an embodiment of the present disclosure, a drug injection device is provided. In addition, embodiments of the present disclosure may be applied to commercially available insulin injection devices and the like.

## Claims

1. A drug injection device comprising:
a base body;
a needle module comprising a needle injected into a user's body and discharging a drug and mounted on the base body;
a cartridge module comprising a reservoir storing the drug discharged to the needle and having a structure attachable to and detachable from the base body; and
a drug discharge assembly disposed between the needle and the reservoir and dispensing the drug to the needle in a preset amount.

2. The drug injection device of claim 1, further comprising
a driving unit for driving the drug discharge assembly,
wherein the cartridge module further comprises a battery supplying power to the driving unit.

3. The drug injection device of claim 2, wherein the cartridge module further comprises a case accommodating the reservoir and the battery as one body.

4. The drug injection device of claim 1, wherein the cartridge module further comprises a connecting member fluidly connecting the reservoir and the drug discharge assembly so that, when the cartridge module is mounted on the base body, the drug in the reservoir flows to the drug discharge assembly.

5. The drug injection device of claim 4, wherein
the drug discharge assembly comprises an insertion portion inserted into the connecting member, and
the connecting member comprises a barrier rib that is penetrable by the insertion portion.

6. A cartridge module replaceably mounted on a drug injection device comprising a base body, a needle module comprising a needle discharging a drug injected into a user's body and mounted on the base body, and a drug discharge assembly dispensing the drug to the needle in a preset amount,
the cartridge module comprising
a reservoir storing the drug discharged to the needle, and a case having a structure accommodating the reservoir therein and attachable to and detachable from the base body.

7. The cartridge module of claim 6, wherein
the cartridge module further comprises a battery supplying power to a driving unit driving the drug discharge assembly, and
the case accommodates the reservoir and the battery as one body.

8. The cartridge module of claim 7, wherein the cartridge module further comprises a connecting member fluidly connecting the reservoir and the drug discharge assembly so that the drug in the reservoir flows to the drug discharge assembly when the cartridge module is mounted on the base body.

9. The cartridge module of claim 8, wherein the connecting member comprises a barrier rib that is penetrable by an insertion portion of the drug discharge assembly.
